Europäisches Patentamt

(19) European Patent Office

Office européen des brevets

(11) Numéro de publication: **0 149 409 B1**

## (12) FASCICULE DE BREVET EUROPEEN

(45) Date de publication de fascicule du brevet:
**10.04.91**

(21) Numéro de dépôt: **84420220.0**

(22) Date de dépôt: **27.12.84**

(51) Int. Cl.⁵: **A61K 7/16, A61K 7/26**

(54) **Agent révélateur de plaque.**

(30) Priorité: **29.12.83 US 566916**

(43) Date de publication de la demande:
**24.07.85 Bulletin 85/30**

(45) Mention de la délivrance du brevet:
**10.04.91 Bulletin 91/15**

(84) Etats contractants désignés:
**AT BE CH DE FR GB IT LI LU NL SE**

(56) Documents cités:
**GB-A- 2 078 109**
**US-A- 4 145 412**
**US-A- 4 376 115**
**US-A- 4 431 628**

**The Merck Index, 9me édition, 1976, page
257, ref.no. 2007: "Chelerythrine";**

(73) Titulaire: **VIPONT PHARMACEUTICAL, INC.**
**220 East Olive Street**
**Fort Collins Colorado 80524(US)**

(72) Inventeur: **Southard, G. Lee**
**1201 Springwood Drive**
**Fort Collins Colorado 80525(US)**

(74) Mandataire: **Dupuis, François**
**Cabinet Laurent et Charras 3, place de
l'Hôtel-de-Ville**
**F-42000 Saint-Etienne(FR)**

## Description

La présente invention est relative une nouvelle composition révélatrice destinée à être utilisée pour l'amélioration des pratiques d'hygiène buccale.

Une plaque dentaire est une structure bien organisée qui se forme sur les surfaces des dents et sur les restaurations. Elle se compose principalement de bactéries entourées par une matrice dérivée essentiellement de la salive et des bactéries elles-mêmes. La plaque diffère d'autres dépôts dentaires friables tels que le matériau alba et les débris de nourriture; en ce sens qu'elle présente une architecture bien définie et qu'elle ne peut pas s'enlever par rinçage avec de l'eau.

C'est un fait bien établi que la plaque dentaire joue un rôle majeur dans l'étiologie des maladies périodontales et des caries. Bien que la façon exacte dont la plaque contribue à ces états pathologiques ne soit pas connue pour le moment, on ne s'en rend pas moins nettement compte qu'une élimination efficace et totale de ces dépôts est absolument essentielle pour l'établissement d'un programme de contrôle dans le plan de traitement de tout malade dentaire. Pour que ce programme soit efficace,il faut que le patient soit incité par une motivation valable à procéder à des techniques de contrôle de plaque quotidien approfondies. Toutefois, cette motivation valable ne peut être obtenue qu'en fixant des buts compréhensibles et accessibles pour le patient. L'expérience a démontré que la plupart des patients ne seraient pas suffisamment incités à pratiquer une bonne hygiène buccale si on leur dit simplement que la plaque est une colonie bactérienne se développant sur leurs dents, que la plaque provoque des affections gingivales et des caries, et qu'il est nécessaire de l'enlever quotidiennement. Or; tout le concept de la nature d'une plaque et des dommages qu'elle cause aux tissus peut être rendu vivant et important pour le patient par la visualisation en montrant à chaque patient sa plaque à pied d'oeuvre et sous le contraste de phase d'un microscope. Le patient peut également observer les zones d'affection gingivale et leur juxtaposition par rapport aux points d'accumulations de plaques. Ces démonstrations visuelles servent à deux buts principaux. premièrement; elles démontrent au patient qu'il a effectivement sur ses dents ces dange reux dépôts de bactéries appelées plaques. Deuxièmement, dans la visualisation au microscope, le patient voit que ces masses d'apparence innocente sont composées de millions de bactéries vivantes de formes diverses. L'expérience a démontré que la technique de la visualisation des plaques crée chez les patients un intérêt réel au sujet des plaques, et un souci évident et apparent en ce qui concerne leur prompte élimination.

Des études récentes ont démontré que la plaque possède un potentiel destructif élevé; et que dans différentes conditions, elle peut déclencher des gingivites et des périodontites, produire des caries dentaires, ou se former en calcul. Il a été également établi qu'une accumulation de plaques qu'on laisse se développer sans l'enlever peut dans de nombreux cas provoquer une gingivite dans un délai de un à vingt et un jours. Il existe également des preuves récentes indiquant que la plaque avec tous ses composants est susceptible de provoquer une réponse allergique dans les tissus souples adjacents.

Cette possibilité virtuelle et relativement importante de destructions a attiré particulièrement l'attention et a eu pour résultat les différents essais entrepris en vue d'éduquer le public pour le contrôle de cette possibilité de destruction. D'une façon générale, on a envisagé pour ce contrôle d'aborder le problème par des lavages de bouche, avec focalisation sur des soins tels que brossage; stimulation, massages, rinçage par pulvérisation et similaires. Toutefois, si ces mesures sont efficaces pour enlever des débris de nourriture et de matières étrangères similaires, elles ne sont par contre pas très efficaces pour enlever les plaques. La formation de plaque est transparente, et donc elle ne se voit pas facilement, particulièrement pour une personne non expérimentée à ce sujet, et il est très fréquent que l'enlèvement se produise surtout de façon accidentelle lors des lavages de bouches.

Afin d'accroitre l'efficacité de la détection des plaques et de leur enlèvement, il a été récemment introduit sur le marché des compositions colorantes, ou appelées compositions révélatrices. Ces compositions révélatrices contiennent des teintures ou des matières colorantes, qui sont destinées à être absorbées par la plaque afin de permettre de distinguer visuellement celle-ci par rapport au reste de la cavité buccale. L'ingrédient colorant actif, dans la plupart de ces compositions révélatrices qu'on trouve dans le commerce, est généralement de l'iode; ou consiste en divers matières colorantes organiques, qui servent d'agent de diagnostic primaire.

Les matières colorantes organiques ont été presque universellement adoptées pour l'utilisation dans les compositions révélatrices se trouvant dans le commerce, en raison du degré d'efficacité élevé de ces matières.

Cependant, dans la plupart des cas de ce genre; ces matières colorantes ont un goût fortement désagréable et déplaisant; qui n'est pas masqué de manière efficace par n'importe quel agent aromatique ou édulcorant connu.

Afin d'obvier à la nécessité d'employer des solutions révélatrices comme moyen de diagnostic, il y a eu une récente introduction sur le marché de systèmes de détection par lumière fluorescente. Ces types de système de détection luminescente sont basés sur des compositions qui sont introductibles dans la cavité buccale; et qui contiennent un ingrédient qui est fluorescent lorsqu'il est activé par une source lumineuse appropriée. On prétend que l'ingrédient fluorescent ou la matière colorante fluorescente est absorbable par la plaque, et que la matière colorante fluorescente ne deviendra fluorescente que dans les zones de formation de plaque et à condition d'être excitée par cette source lumineuse appropriée. Mais dans la majorité des cas, la matière colorante devient fluorescente a la même couleur que l'émail, et en conséquence la plaque ne peut pas être décelée avec la facilité désirable. Par ailleurs ,le prix d'achat de ces systèmes et les difficultés éprouvées lors de l'utilisation de ces systèmes se sont généralement opposées à une diffusion de leur emploi à grande échelle.

Ainsi selon l'invention, il est revendiqué une composition pour la visualisation de la formation de plaques dans la cavité buccale et rendre cette formation de plaques visibles à l'oeil nu sous lumière ultraviolette à grande longueur d'onde, caractérisée en ce que cette composition comprend un sel de sanguinarine choisi dans le groupe comprenant le chlorure de sanguinarine, du nitrate de sanguinarine, du sulfate de sanguinarine et comprenant de plus un mélange de sels précipités à partir d'extraits de plantes choisies dans le groupe comprenant la Sanguinaria canadensis, Macleaya cordata, Cordyalis sevctvozii, C. Tedebouni, Chelidonium, majus et mélanges de celles-ci, et en ce que le sel de sanguinarine est présent en une quanitité de 0, 001 à 1 % en poids, et ce mélange de sels est présent en une quantité de 0,003 à 3 % en poids.

Il a été constaté que les sels de sanguinarine révèlent la plaque tout aussi bien que les deux agents révélateurs standards, l'erythosine et la fluoroscéine. Mais il a été constaté en outre que la sanguinarine était retenue dans ou sur les dépôts de plaque durant un laps de temps bien plus prolongé que les agents révélateurs standards; et on sait que la sanguinarine possède des propriétés anti-microbiennes.

La durée de rétention plus prolongée de la sanguinarine sur les dépôts de plaque en fait un révélateur idéal pour l'utilisation dans les soins dentaires, étant donné que la sanguinarine permet des lavages dentaires durant la période de révélation tout en maintenant son action anti-bactérienne; ce qui est particulièrement important dans le cas de détartrage ultrasonique, ou l'aéralisation de dépôts buccaux constitue un risque professionnel pour les dentistes et les hygiénistes dentaires. Les compositions renfermant de la sanguinarine en tant qu'agent révélateur peuvent également être utilisées par les consommateurs pour self-révélation de plaque à la maison, dans un but de contribution à l'hygiène buccale.

La sanguinarine peut être incorporée de plusieurs façons dans une composition révélatrice. La méthode la plus commune consiste à incorporer la sanguinarine dans une composition de lavage de bouche utilisée pour rincer la bouche avant de vérifier l'existence de dépôts de plaque. En variante, la sanguinarine peut être incorporée dans un biscuit test ou autre produit alimentaire mastiquable tel que bonbon ou chewing gum comme excipient pour le diagnostic de la plaque. La sanguinarine peut également être incorporée dans du dentifrice, afin de permettre au sujet de procéder à des observations pendant qu'il se brosse les dents.

En formulant les préparations appropriées pour l'usage précité, on peut comprendre, si on le désire, un ou plusieurs additifs qui sont utiles pour d'autres applications. On peut par exemple utiliser, dans des buts divers, des agents de brillantage, des solvants en pulvérisation, ou des agents mouillants, etc...On peut utiliser pratiquement tout produit connu en eau dentifrice, pâte dentifrice, poudre dentifrice ou autre formule utile pour le diagnostic ou le traitement thérapeutique de surfaces externes du corps et de la cavité buccale.

Le chlorure de sanguinarine a été comparé à deux matières colorantes révélatrices standards, l'érythrocine et la fluoroscéine de sodium. A des intervalles hebdomadaires, des volontaires en bon état de santé ont été soumis à une période de l2 - 24 heures sans hygiène buccale. Les sujets ont alors utilisé un des produits suivants : érytrocine, fluoroscéine de sodium; ou chlorurre de sanguinarine. Les sujets se sont rincés deux fois avec 15 ml du produit de rinçage tandisque le'érytrocine et la fluoroscéine de sodium ont été utilisées selon la pratique courante : La couleur a été évaluée à la lumière ambiante après l'érythrocine, à la lumière Plak pour la fluoroscéine de sodium; et à la lumière fluorescente ultra-violette onde longue pour la sanguinarine La couleur a été repérée au point de vue emplacement; quantité; et surface.

Tous les agents ont coloré des dépôts dentaires friables aux marges gingivales et à la surface dorsale de la langue. L'érythrocyne a également coloré les gencives et d'autres tissus souples, alors que ce n'a pas été le cas pour la fluoroscéine et la sanguinarine. Les valeurs moyennes de plaque ont été mesurées pour tous les révélateurs. Le tableau montre que le liquide dentifrice au chlorure de sanguinarine a révélé la plaque de manière efficace, et que la révélation durait sensiblement plus longtemps que pour les deux autres matières colorantes révélatrices.

TABLEAU I    Constatation visuelle

Agent actif    repérage d'aire de plaque moyenne

point de départ    1 heure après

| Agent actif | point de départ | 1 heure après |
|---|---|---|
| Erythrocine | 2,86 + 0,43 | 2,18 + 0,5 |
| Fluorescéine de sodium | 2,84 + 0,22 | 1,74 + 0,45 |
| Chlorure de sanguinarine | 2,87 + 0,23 | 2,86 + 0,26 |

L'évaluation quantitative de la sanguinarine dans la plaque et la salive à l'aide de chromatographie liquide à haute performance a fait apparaitre des niveaux dans la plaque beaucoup plus élevés que les concentrations inhibitrices minimum in vitro, comme montré au tableau II.

TABLEAU II

Rétention de Plaque in Vivo de Sanguinarine (ug/g de Plaque Humide)

Durée d'essai, minutes

| Sujet | 15 | 30 | 45 | 60 | 90 | 120 |
|---|---|---|---|---|---|---|
| 1 | 26 | – | 22 | – | 26 | – |
| 1A | 110 | – | 27 | – | 30 | – |
| 2 | 29 | – | 27 | – | 31 | – |
| 2A | 79 | – | 56 | – | 46 | – |
| 3 | 111 | – | 37 | – | 36 | – |
| 3A | 160 | – | 34 | – | 36 | – |
| 4 | – | 96 | – | 56 | 51 | – |
| 5 | – | 46 | – | 46 | 46 | 27 |

Les niveaux de sanguinarine dans la salive; comme montré au Tableau III, ont été suffisamment élevés pour exercer un effet anti-glycolitique sur la salive. Ceci indiquerait que la rétention de la sanguinarine de la plaque peut être la cause de l'efficacité clinique de la sanguinarine dans les essais de plaque, et que cette plaque peut servir de réservoir pour la sanguinarine.

## TABLEAU III

### Rétention Salive in Vivo de Sanguinarine (ug/ml de salive)

| Sujet | Durée d'essai; minutes | | |
|---|---|---|---|
| | 15 | 45 | 60 |
| 6 | 1,13 | 0,92 | 0,45 |
| 7 | 1,47 | 0,75 | 0,60 |
| 26 | 0,96 | 0,79 | 1,00 |
| 20 | 0,80 | 0,57 | 0,42 |
| 27 | 0,90 | 0,90 | 0,80 |
| 21 | 0,47 | 0,17 | 0,22 |
| 28 | 2,63 | 1,25 | 0,54 |
| 22 | 0,50 | 0,29 | 0,18 |
| 24 | 1,05 | 0,85 | 0,80 |

Description des mises en eouvres préférées :

Il a été constaté que le chlorure de sanguinarine et ses sels apparentés sont utiles pour le contrôle de la plaque dentaire. Les sels de sanguinarine dont on a constaté l'utilité dans des agents révélateurs de plaque comprennent les sels de chlorure; de nitrate et de sulfate, ainsi que tous les mélanges susceptibles d'être précipités ou purifiés à partir des extraits de Sanguinaria canadensis et autres membres de la famille des Papavéracées.

Les sels de saguinarine qui ont été trouvés utiles comme agent révélateurs de plaque sont des sels de sanguinarine, soit sous forme d'isolats purs; soit sous forme de mélanges; de sels benzophenanthridines tels que nitrate de sanguinarine; sulfate de sanguinarine, Sanguiritine, et tous les autres mélanges pouvant être obtenus par précipitation à partir des extraits de Sanguinaria canadensis, Madleaya cordate, carydalis sevctvozii; C. lebebouni; chelidonium majusm et autres membres des Papaveracées.

## EXEMPLE I

On a mis au point une formule de liquide de lavage de bouche destiné à être utilisé comme agent révélateur. Le produit contenait les ingrédients suivants :

| | | |
|---|---|---|
| Alcool ethylique | 10,00 | % |
| Extrait de sanguinarine | 0,03 | % |
| Acide citrique | 0,03 | % |
| Aromate (essence de cannelle) | 0,25 | % |
| Polysorbate 80 | 0,60 | % |
| Glycérine | 3,46 | % |
| Eau déionisée | 85,23 | % |
| Poloxamer 40 % | 0,10 | % |
| Saccharine de sodium | 0,10 | % |
| Chlorure de zinc | 0,20 | % |

Ceci a été le liquide de lavage buccal utilisé pour les essais décrits aux Tableaux I, II et III.

EXEMPLE II

On a mis au point une formule de liquide de lavage de bouche destiné à être utilisé comme agent révélateur; en substituant 0;10 % des sels mixtes obtenus par précipitation à partir de Sanguinaria canadensis au chlorure de sanguinarine ci-avant. Ce liquide de lavage buccal, utilisé dans une quantité de 15 ml environ pendant 15 secondes dans la bouche; assure une révélation de plaque acceptable dans la bouche sous lumière-ultra-violette onde longue (environ 365 mm). `

Les liquides de lavage buccal destinés à être utilisés comme agents révélateurs de plaque peuvent incorporer de 0,001 à 1 % de sel de sanguinarine pur (chlorure; nitrate; sulfate) et de 0;003 % environ d'alcaloïde benzophenanthridine mixte (34 % de sanguinarine en poids).

Les compositions de liquide de lavage buccal dont l'utilité a été constatée pour la pratique de la présente invention comprennent généralement une solution eau/alcool éthylique et; de manière facultative, d'autres ingrédients tels que des aromates, des édulcorants et des humectants. Le liquide de lavage peut également contenir des agents émulsifiants pour aider à la pénétration de la plaque. L'agent émulsifiant est généralement présent dans des quantités de 0 à 12 % environ en poids, avec des agents aromatisants et colorants facultatifs.

Des agents émulsifiants appropriés sont ceux qui sont raisonnablement stables et qui forment des mousses sur une gamme étendue de pH, c'est-à-dire par exemple des détergents synthétiques organiques non saponifiés; non ioniques; cationiques et amphotériques.

Les détergents synthétiques non ioniques susceptibles d'être utilises avec les compositions de lavage buccal de la présente invention peuvent être définis dans les grandes lignes comme des composés produits par la condensation d'un groupe d'oxyde hydrophile avec un composé hydrophobe organique pouvant être de nature aliphatique ou aromatique. La longueur du radical hydrophile ou polyoxyalkylène qui est condensé avec tout groupe hydrophobe particulier quelconque peut être facilement réglée de manière à donner un composé soluble dans l'eau ayant le degré d'équilibre désiré entre les éléments hydrophiles et hydrophobes.

Les détergents synthétiques cationiques dont l'utilité a été constatée dans les compositions de liquide dentifrice de la présente invention peuvent être définis dans les grandes lignes comme des compositions ammoniacales quaternaires comportant une chaîne alkyle longue contenant d'environ 8 à environ 18 atomes de carbone, tels que du lauryltriméthylammonium, du chlorure cétylique de pyridinium; du bromure cétylique de triméthylammonium; du chlorure de di-isobutylphénoxyéthyldiméthylbenzylammonium; nitrure de cocoalkyltriméthylammonium; fluorure cétylique de pyridinium et similaires.

Les détergents synthétiques amphotériques dont l'utilité a été constatée dans la présente invention peuvent être définis dans les grandes lignes comme des dérivés aliphatiques secondaires et tertiaires dans lesquels le radical aliphatique peut être linéaire ou ramifié, et dans ce radical, un des substituants aliphatiques contient d'environ 8 à environ 18 atomes de carbone; et un de ces substituants contient un groupe anionique sobulilisant à l'eau; tels que des carboxylates, des sulphonates; des sulfates; des phosphates ou des phosponates.

Le liquide de lavage buccal destiné à être utilisé comme agent révélateur peut également contenir des agents aromatiques tels que de l'essence de wintergreen, (salicylate de méthyle); essence de menthe, essence de sassafras; et essence d'anis. Les agents aromatisants peuvent être prévus à des niveaux allant d'environ 0,05 % à environ 2 % en poids.

Les liquides de lavage buccal peuvent également contenir des agents édulcorants, tels que saccharine, dextrose, et aspartame; levulose. Les agents édulcorants sont utilisés à des niveaux allant d'environ 0,05 % à environ 2 % en poids.

Les compositions révélatrices de la présente invention peuvent également être incorporées dans des pâtes ou poudres pour applications locales, ou incorporées sous forme de produits alimentaires mastiquables pouvant être mastiqués par le patient de façon à assurer l'application de la composition révélatrice.

EXEMPLE III

Une pâte dentifrice est obtenue en mélangeant ensemble les ingrédients suivants dans les proportions indiquées.

| | |
|---|---|
| Métaphosphate de sodium insoluble | 26,60 % |
| Phosphate de dicalcium | 26,60 % |
| Gomme | 1,40 % |
| Aromate | 1,60 % |
| Sulfate laurylique de sodium | 1,10 % |
| Glycérol (40,7 %) et eau (1,0 %) | 41,70 % |
| Sulfate de sanguinarine | 1,00 % |

Les dents du patient ont été brossées avec cette pâte; et ont été exposées ensuite à la lumière ultra-violette onde longue. La plaque sur les dents se détachait de façon très marquée; en relief bien visible, par rapport aux surfaces propres:et saines adjacentes.

EXEMPLE IV

Une pâte dentifrice est obtenue en mélangeant ensemble les ingrédients suivants, dans les proportions indiquées :

| | |
|---|---|
| Hydrate d'aluminium microcristallin | 91,25 % |
| Hydrate d'aluminium (maille 325) | 5,00 % |
| Matière aromatisante | 0,60 % |
| Saccharine, soluble | 0,25 % |
| Fluorure de sodium | 0,10 % |
| Sulfoacétate laurylique de sodium | 2,30 % |
| Chlorure de sanguinarine | 0,50 % |

Les sels de sanguinarine peuvent également être incorporés dans des produits alimentaires mastiquables tels que chewing gum; confits, bonbons, ou biscuits, pouvant être mastiqués par le patient de manière à assurer l'application de sels de sanguinarine sur les dents afin de permettre la révélation de la plaque.

**Revendications**

1. Composition pour la visualisation de la formation de plaques dans la cavite buccale et rendre cette formation de plaques visibles à l'oeil nu sous lumière ultraviolette à grande longueur d'onde, caractérisée en ce que cette composition comprend un sel de sanguinarine choisi dans le groupe comprenant le chlorure de sanguinarine, du nitrate de sanguinarine, du sulfate de sanguinarine, et comprenant de plus un mélange de sels précipités à partir d'extraits de plantes choisies dans le groupe comprenant la Sanguinaria canadensis, Macleaya cordata, Cordyalis sevctvozii, C. Tedebouni, Chelidonium, majus et mélanges de celles-ci, et en ce que le sel de sanguinarine est présent en une quantité de 0, 001 à 1 % en poids, et ce mélange de sels est présent en une quantité de 0,003 à 3 % en poids.

2. Composition selon la revendication 1, caractérisée en ce que cette composition est un rinçage buccal.

3. Composition selon la revendication 1, caractérisée en ce que le sel de sanguinarine est du chlorure de sanguinarine.

4. Composition selon la revendication 1, caractérisée en ce que le sel le sanguinarine est un mélange de sels de benzophénanthridine extraits de la Sanguinaria Canadensis.

5. Composition selon la revendication 1, caractérisée en ce que cette composition est une pâte dentifrice.

6. Composition selon la revendication 1, caractérisée en ce que cette composition est une poudre dentifrice.

**Claims**

1. Composition for the visualization of the formation of plates in the buccal cavity and to make this formation of plates visible with the naked eye under the ultra-violet light with great wave length, characterized in that this composition includes a sanguinarine salt selected in the group including sanguinarine chlorid, sanguinarine nitrate, sanguinarine sulphate, and comprising moreover a mixture of salts precipitated from plant extracts selected in the group comprising the Sanguinaria canadensis, the Macleaya cordata, the Cordialis sevctvozii, the C. Tedebouni, the Chelidonium majus, and mixtures of same, and in that the sanguinarine salt is present in a quantity of 0,001 to 1 wt.%, the salt mixture being present in a quantity of 0.003 to 3 wt.%.

2. Composition as claimed in Claim 1, characterized in that this composition is a buccal rinsing.

3. Composition as claimed in Claim 1, characterized in that the sanguinarine salt is the sanguinarine chlorid.

4. Composition as claimed in Claim 1, characterized in that the sanguinarine salt is a mixture of benzophénantridine salts extracted from the Sanguinaria Canadensis.

5. Composition as claimed in Claim 1, characterized in that this composition is a tooth paste.

6. Composition as claimed in Claim 1, characterized in that this composition is a tooth powder.

**Ansprüche**

1. Verbundstoff für die Sichtigkeit der Bildung der Zahnplatten in der Mundhöhle, damit diese Bildung von Zahnplatten mit blossem Auge unter Ultraviolettlicht mit grösser Wellenlange sichtbar wird, dadurch gekennzeichnet, dass dieser Verbundstoff ein Sanguinarine-Salz enthält, welches aus einer Gruppe gewählt wird, die aus den folgenden Chemikalien : Sanguinarine-Chlorid, Sanguinarine-Nitrat, Sanguinarine-Sulfat besteht, und die darüber hinaus ein Gemisch von Niederschlagsalzen aus Pflanzextrakten auch entählt, die aus der die Sanguinaria canadensis, Macleaya cordata, Cordyalis sevctvozii, C. Tedebouni, Chelidonium majus und Gemische derselben umfassenden Gruppe gewählt sind, und dass das Sanguinarine-Salz in einer Menge von 0,001 bis 1 %-Gew. vorliegt, wobei das Salzgemisch in einer Menge von 0,003 bis 3 %-Gew. auch vorliegt.

2. Verbundstoff nach Anspruch 1, dadurch gekennzeichnet, dass dieser Verbundstoff ein Mundabspülen ist.

3. Verbundstoff nach Anspruch 1, dadurch gekennzeichnet, dass das Sanguinarine-Salz Sanguinarine-Chlorid ist.

4. Verbundstoff nach Anspruch 1, dadurch gekennzeichnet,dass Sanguinarine-Salz ein Gemisch von aus Sanguinaria canadensis extrahierten Benzophenantridine-Salzen ist.

5. Verbundstoff nach Anspruch 1, dadurch gekennzeichnet, dass dieser Verbundstoff eine Zahnpaste ist.

6. Verbundstoff nach Anspruch 1, dadurch gekennzeichnet, dass dieser Verbundstoff ein Zahnpulver ist.